# EUROPEAN PATENT APPLICATION

(11) **EP 3 006 533 A1**
(43) Date of publication of application: **13.04.2016**
(21) Application number: 15188636.3
(22) Date of filing: 06.10.2015
(51) Int. Cl.: C09J 7/02

(54) **PRESSURE SENSITIVE ADHESIVE TAPE**

(30) Priority: 06.10.2014 JP 2014205372; 30.01.2015 JP 2015017819
(71) Applicant: Lintec Corporation, Tokyo 173-0001 (JP)
(72) Inventor: MIYATAKE, Yusuke, Tokyo, Tokyo 173-0001 (JP); MIYAZAKI, Kentaro, Tokyo, Tokyo 173-0001 (JP); TAYA, Naoki, Tokyo, Tokyo 173-0001 (JP); KONDO, Takeshi, Tokyo, Tokyo 173-0001 (JP)
(74) Representative: Raynor, Stuart Andrew

(57) **Abstract**

[Problems] There is provided a pressure sensitive adhesive tape which can reduce the amount of migrated components from the pressure sensitive adhesive tape to an adherend while appropriately ensuring the attaching property to the adherend.

[Solution] A pressure sensitive adhesive tape comprising a base material and a pressure sensitive adhesive layer provided on one surface of the base material, wherein the pressure sensitive adhesive layer is formed of a pressure sensitive adhesive composition that contains a block copolymer of an acrylic-based monomer as the base polymer, wherein the total amount of an unreacted monomer raw material contained in the pressure sensitive adhesive layer is 100 ppm or less.

## Description

### [Technical Field]

The present invention relates to a pressure sensitive adhesive tape and specifically to a pressure sensitive adhesive tape that has a reduced amount of migrated components to an adherend and is suitably used in the fields, such as food and medical fields, for which high quality and sophisticated management system are required. In the present description, the concept of a "tape" encompasses the concepts of a "sheet" and a "film."

### [Background Art]

Pressure sensitive adhesive tapes are employed in various uses. It may be desired, not only that the pressure sensitive adhesion to an adherend is merely high, but also that a so-called adhesive deposit, i.e., a phenomenon that the substances which constitute the pressure sensitive adhesive layer of the pressure sensitive adhesive tape are left on the adherend, is unlikely to occur when the pressure sensitive adhesive tape has been peeled off from the adherend.

In order to provide such a pressure sensitive adhesive tape leaving less adhesive deposit, Patent Literature 1 describes a pressure sensitive adhesive tape comprising a base material and a pressure sensitive adhesive layer formed on at least one surface of the base material. The pressure sensitive adhesive layer is formed by applying a pressure sensitive adhesive composition that contains: (A) 100 weight parts of a (meth)acrylic-based copolymer obtained by polymerizing a monomer mixture containing (a) 83 to 99 wt% of an alkyl (meth)acrylate of which the carbon number of an alkyl group is 5 to 12, (b) 1.0 to 3.0 wt% of a carboxyl group-containing monomer, and (c) 0 to 15 wt% of other polymerizable monomers; (B) 0.5 to 15 weight parts of a polymerized rosin-based resin as a tackifier resin B; (C) 1 to 15 weight parts of a polyester-based compound as a plasticizer; and (D) 2.0 to 5.0 weight parts of a carbodiimide-based compound as a curing agent. As used in the present description, the term "(meth)acrylic acid" means both of acrylic acid and methacrylic acid. The same applies to other similar terms.

### [Prior Art Literature]

### [Patent Literature]

[Patent Literature 1] JP2014-224208A

### [Summary of the Invention]

### [Problems to be solved by the Invention]

The pressure sensitive adhesive tape as described in Patent Literature 1 is said to be suitably usable as a specific tape, such as a masking tape for painting works and a tape for fixing a vinyl sheet to a wall or the like when load carriage operation is performed. However, in the fields, such as food and medical fields, for which high quality and sophisticated management system are required, the amount of migrated components from the pressure sensitive adhesive layer to an adherend may have to be reduced to about 100 ppm or less, thereby to suppress the pollution of the adherend and/or to allow the constitutional components of the pressure sensitive adhesive layer to be unlikely to remain on the adherend when the pressure sensitive adhesive tape has been peeled off from the adherend. The pressure sensitive adhesive tape as described in Patent Literature 1 may be difficult to respond to such a request as the above while appropriately ensuring the attaching property to an adherend.

The present invention has been created in view of such actual circumstances as described above, and objects of the present invention include providing a pressure sensitive adhesive tape which can reduce the amount of migrated components from the pressure sensitive adhesive tape to an adherend while appropriately ensuring the attaching property to the adherend.

### [Means for solving the Problems]

The present invention provided to achieve the above objects is as follows:
(1) A pressure sensitive adhesive tape comprising a base material and a pressure sensitive adhesive layer provided on one surface of the base material, wherein the pressure sensitive adhesive layer is formed of a pressure sensitive adhesive composition that contains a block copolymer of an acrylic-based monomer as a base polymer, wherein a total amount of an unreacted monomer raw material contained in the pressure sensitive adhesive layer is 100 ppm or less.
(2) The pressure sensitive adhesive tape as described in the above (1), wherein the block copolymer contains a triblock copolymer as a main component.
(3) The pressure sensitive adhesive tape as described in the above (2), wherein the triblock copolymer satisfies at least one of a condition that a polystyrene equivalent weight-average molecular weight (Mw) is 30, 000 or more and 300, 000 or less and a condition that a molecular weight distribution (Mw/Mn) is 5.0 or less.
(4) The pressure sensitive adhesive tape as described in any one of the above (1) to (3), wherein a peel strength in 180° peeling from a SUS plate (SUS304 #600) is 0.1 N/25 mm or more when measured in accordance with JIS Z0237: 2009.
(5) The pressure sensitive adhesive tape as described in any one of the above (1) to (4), wherein a ball stack value at a slope angle of 30° is 10 or more when measured in accordance with JIS Z0237: 2009.
(6) The pressure sensitive adhesive tape as described in any one of the above (1) to (5), wherein a holding time is 70,000 seconds or more when a holding power test is performed under conditions of a sample attaching area of 25 mm×25 mm, a load of 1 kg, and a temperature of 40°C in accordance with JIS Z0237: 2009.

The pressure sensitive adhesive tape as described in any one of the above (1) to (6), wherein the pressure sensitive adhesive layer is produced by extrusion molding.

### [Advantageous Effect of the Invention]

According to the present invention, there is provided a pressure sensitive adhesive tape which can reduce the amount of migrated components from the pressure sensitive adhesive tape to an adherend while appropriately ensuring the attaching property to the adherend.

### [Embodiments for Carrying out the Invention]

Hereinafter, embodiments of the present invention will be described.

The pressure sensitive adhesive tape according to an embodiment of the present invention comprises a base material and a pressure sensitive adhesive layer provided on one surface of the base material. The pressure sensitive adhesive tape may further comprise a release sheet provided on a surface of the pressure sensitive adhesive layer opposite to the surface facing the base material.

The pressure sensitive adhesive tape according to an embodiment of the present invention is configured such that the pressure sensitive adhesive layer of the pressure sensitive adhesive tape is formed of a pressure sensitive adhesive composition that contains a block copolymer of an acrylic-based monomer as the base polymer.

In general, when the pressure sensitive adhesive composition for forming the pressure sensitive adhesive layer contains a polymer of an acrylic-based monomer as the base polymer, polymerization of the acrylic-based monomer for obtaining such a polymer is ordinarily performed by free radical polymerization using a polymerization initiator such as azobisisobutyronitrile (AIBN). In this case, however, the molecular weight distribution of the obtained polymer tends to have a broad peak, and an unreacted monomer raw material of several hundred ppm may remain.

In contrast, when the pressure sensitive adhesive composition for forming the pressure sensitive adhesive layer contains a block copolymer of an acrylic-based monomer as the base polymer, block copolymerization of the acrylic-based monomer for obtaining such a block copolymer is performed by living anionic polymerization. Since the molecular weight distribution of the copolymer obtained by living anionic polymerization tends to have a sharper peak than that of a polymer obtained by free radical polymerization, an unreacted monomer raw material is unlikely to remain in the block copolymer of an acrylic-based monomer.

Specifically, the pressure sensitive adhesive layer of the pressure sensitive adhesive tape according to an embodiment of the present invention is configured such that the total amount of an unreacted monomer raw material contained in the pressure sensitive adhesive layer is 100 ppm or less. In the present description, the total amount of an unreacted monomer raw material contained in the pressure sensitive adhesive layer refers to a value as measured in the following manner. An evaluation sample is obtained by cutting the pressure sensitive adhesive tape so that the exposed surface of the pressure sensitive adhesive layer has a surface area of 20 cm². The evaluation sample is enclosed in an ampule, which is then heated at 120°C for 30 minutes in Purge & Trap GCMass ("JHS-100A" available from Japan Analytical Industry Co., Ltd.) to sample gasses, which are thereafter introduced into GCMass ("TurboMass" available from PerkinElmer), and with reference to a calibration curve prepared using n-decane, the amount of sampled monomers is obtained as an n-decane equivalent amount (unit: ppm), which is to be the total amount of an unreacted monomer raw material.

When the total amount of an unreacted monomer raw material contained in the pressure sensitive adhesive layer is 100 ppm or less as the above, the amount of migrated components from the pressure sensitive adhesive layer to an adherend will be small, so that the problems of pollution of the adherend and adhesive deposit are unlikely to occur. In the present description, the amount of migrated components from the pressure sensitive adhesive layer to an adherend refers to an amount measured by a test method performed in accordance with "the basic rules necessary for testing migration of the constituents of plastic materials and articles intended to come into contact with foodstuffs" (Directive 82/711/EEC, 97/48/EC). Specifically, a test sample is obtained by removing a release sheet from the pressure sensitive adhesive tape and attaching the pressure sensitive adhesive tape to a frame-shaped jig for exposing a predetermined size (surface area of 3 dm²) of the surface of the pressure sensitive adhesive layer. Subsequently, the test sample is stationarily placed at 20°C for 24 hours so that the surface of the pressure sensitive adhesive layer of the pressure sensitive adhesive tape is in contact with 150 ml of an extraction solvent (95 vol% ethanol) . Thereafter, only the extraction solvent is dried at 20°C, and drying operation is further performed at 120 °C for 30 minutes. The weight of residue after drying is measured, and the measured value is to be the amount of migrated components.

As previously described, the block copolymer of an acrylic-based monomer has a molecular weight distribution with a relatively sharp peak. Therefore, in the pressure sensitive adhesive layer formed of the pressure sensitive adhesive composition which contains the block copolymer of an acrylic-based monomer as the base polymer, the content of low molecular weight components (the "low molecular weight components" as used in the present description refer collectively to an unreacted monomer raw material and an oligomer of which the degree of polymerization is low and the molecular weight is about 10,000 or less), which can relatively easily move in the pressure sensitive adhesive layer, may possibly be small. As such, the amount of migrated components to an adherend appears to be small in the pressure sensitive adhesive layer of the pressure sensitive adhesive tape according to an embodiment of the present invention.

It is preferred that the above block copolymer of an acrylic-based monomer contains a triblock copolymer as the main component. Specific examples of such a triblock copolymer include polymethyl methacrylate (PMMA)-polybutyl acrylate (PBA)-polymethyl methacrylate (PMMA) and polymethyl methacrylate (PMMA)-poly2-ethylhexyl acrylate (2EHA)-polymethyl methacrylate (PMMA). The triblock copolymer has a structure in which relatively hard segments (hard segments) of PMMA capable of pseudo-crosslinking are linked with both ends of a relatively soft segment (soft segment) of PBA or 2EHA.

It is also possible to control the cohesive strength of the triblock copolymer as a whole, such as by adjusting the type and the length of the soft segments, the quantitative balance between the soft segments and the hard segments, etc. Therefore, owing to the feature of the pressure sensitive adhesive tape according to an embodiment of the present invention that the pressure sensitive adhesive of the pressure sensitive adhesive layer contains a triblock copolymer as the main component, it is possible to have excellent attaching properties to an adherend (including removability from an adherend).

When the temperature of the triblock copolymer is high, each polymer may tend to solely exist, but the triblock copolymer can be designed such that, when the temperature is around the room temperature, the segments located at both ends and capable of pseudo-crosslinking interact with segments of other polymer capable of pseudo-crosslinking, thereby to have a three-dimensional collective structure as a whole. Therefore, owing to the feature of the pressure sensitive adhesive tape according to an embodiment of the present invention that the pressure sensitive adhesive composition for forming the pressure sensitive adhesive layer contains a triblock copolymer as the base polymer, a pressure sensitive adhesive layer can be obtained which has excellent physical properties (such as storage elastic modulus and tensile strength) after molding due to the three-dimensional collective structure while maintaining good flowability during injection molding or extrusion molding. Explaining from another aspect the change in physical properties due to temperature, at the stage where the triblock copolymer is put into the hopper of a molding machine, autohesion of pellets may not readily occur, whereas at the stage where the triblock copolymer is molten, the flowability can be enhanced, so that troubles are unlikely to occur when the pressure sensitive adhesive layer is formed by molding.

It is preferred that the above triblock copolymer satisfies at least one of a condition that the polystyrene equivalent weight-average molecular weight (Mw) is 30,000 or more and 300,000 or less and a condition that the molecular weight distribution (Mw/Mn) is 5.0 or less.

In the present description, the value of the polystyrene equivalent weight-average molecular weight (Mw) refers to a value measured as a standard polystyrene equivalent value by the gel permeation chromatography (GPC) using a solvent of tetrahydrofuran (THF). Specifically, the measurement is to be performed using a GPC measurement apparatus ("HLC-8220GPC" available from TOSOH CORPORATION) under the following conditions:
Columns: TSKgel GMHXL → TSKgel GMHXL → TSKgel 2000HXL
Injection amount: 80 µl
Measurement temperature: 40°C
Flow rate: 1 ml/min
Detector: differential refractometer
Sample concentration: 1% (w/v)

According to the feature that the polystyrene equivalent weight-average molecular weight (Mw) of the above triblock copolymer is 30,000 or more and 300,000 or less, the pressure sensitive adhesive layer formed of the pressure sensitive adhesive composition, which contains such a triblock copolymer as the base polymer, may readily have both of enhanced adhesion to an adherend and high cohesive strength of the pressure sensitive adhesive layer. Therefore, the pressure sensitive adhesive tape according to an embodiment of the present invention, which comprises such a pressure sensitive adhesive layer, has an excellent attaching property to an adherend. In view of stably enhancing the adhesion of the pressure sensitive adhesive tape to an adherend and the cohesive strength of the pressure sensitive adhesive layer, the polystyrene equivalent weight-average molecular weight (Mw) of the above acrylic block copolymer may preferably be within a range from 30,000 to 300,000, more preferably within a range from 40,000 to 250,000, and highly preferably within a range from 50,000 to 200,000.

According to the feature that the molecular weight distribution (Mw/Mn) of the above triblock copolymer is 5.0 or less, it can be more stably realized to reduce the amount of low molecular weight components contained in the triblock copolymer. In view of further stably reducing the above amount of low molecular weight components, the molecular weight distribution (Mw/Mn) of the above triblock copolymer may preferably be 1.0 or more and 5.0 or less, more preferably 1.0 or more and 3.0 or less, and particularly preferably 1.0 or more and 2.0 or less. When the condition with regard to the range of the above polystyrene equivalent weight-average molecular weight (Mw) is also satisfied, the amount of low molecular weight components contained in the triblock copolymer can be particularly stably reduced. Therefore, by satisfying the condition with regard to the above molecular weight distribution (Mw/Mn), and preferably by also satisfying the condition with regard to the range of the above polystyrene equivalent weight-average molecular weight (Mw), it may be more stably realized, in the pressure sensitive adhesive tape according to an embodiment of the present invention, to reduce the amount of migrated components from the pressure sensitive adhesive tape to an adherend.

In the pressure sensitive adhesive tape according to an embodiment of the present invention, it is preferred that the peel strength in 180° peeling from a SUS plate (SUS304 #600) is 0.1 N/25 mm or more when measured in accordance with JIS Z0237 : 2009 (ISO 29862-29864: 2007). When a pressure sensitive adhesive layer is formed of a pressure sensitive adhesive composition that contains, as the base polymer, an acrylic-based polymer polymerized by radical polymerization as in the prior art, the pressure sensitive adhesive layer may contain several hundred ppm of low molecular components. Such low molecular components can function, in the pressure sensitive adhesive layer, as components that enhance the pressure sensitive adhesion of the pressure sensitive adhesive tape to an adherend, like a so-called tackifier. Therefore, a pressure sensitive adhesive tape comprising a pressure sensitive adhesive layer formed of a pressure sensitive adhesive composition that contains, as the base polymer, a block copolymer of an acrylic-based monomer, like the pressure sensitive adhesive tape according to an embodiment of the present invention, can have a tendency that the attaching property, in particular the pressure sensitive adhesion, to an adherend deteriorates.

When the above peel strength in 180° peeling is 0.1 N/25 mm or more, however, the pressure sensitive adhesive tape according to an embodiment of the present invention may appropriately ensure the attaching property to an adherend, which may be preferred. In view of more stable realization of appropriately ensuring the attaching property of the pressure sensitive adhesive tape to an adherend, the above peel strength in 180° peeling may more preferably be 0.5 N/25 mm or more, further preferably 1.0 N/25 mm or more, and particularly preferably 3.0 N/25 mm or more.

The upper limit of the above peel strength is not provided, but should be set in accordance with the intended use. Examples of the upper limit include, but are not limited to, being 50 N/25 mm or less (when having no removability) and being 30 N/25 mm or less (when having removability).

The pressure sensitive adhesive tape according to an embodiment of the present invention may preferably be such that a ball stack value at a slope angle of 30° is 10 or more when measured in accordance with JIS Z0237 : 2009 and the J. Dow method. According to the feature that the above ball stack value is 10 or more, the stickiness of the surface of the pressure sensitive adhesive layer of the pressure sensitive adhesive tape may be more appropriate, thereby improving the attaching property of the pressure sensitive adhesive tape to an adherend. In view of more stable realization of appropriately ensuring the attaching property of the pressure sensitive adhesive tape to an adherend, the above ball stack value may more preferably be 12 or more, and further preferably 15 or more.

The pressure sensitive adhesive tape according to an embodiment of the present invention may preferably be such that a holding time is 70,000 seconds or more when a holding power test is performed under conditions of a sample attaching area of 25 mm×25 mm, a load of 1 kg, and a temperature of 40°C in accordance with JIS Z0237: 2009. The holding time is a period of time from when the test was started to when the pressure sensitive adhesive tape comes off and a weight as the applied load drops off. According to the feature that the holding time is 70, 000 seconds or more, when the pressure sensitive adhesive tape is attached to an adherend, the problem of running off of the constitutional components of the pressure sensitive adhesive layer is less likely to occur, and the pressure sensitive adhesive tape may readily have an appropriate attaching property to the adherend.

Material of the base material of the pressure sensitive adhesive tape according to an embodiment of the present invention is not limited. The base material may be composed of a resin-based sheet that contains a resin-based material as the main component, or may also be composed of a paper-based material.

Examples of resin components of the resin-based sheet include: olefin-based polymer such as polyethylene, polypropylene and ethylene-(meth)acrylic acid copolymer; ester-based polymer such as polyethylene terephthalate and polybutylene terephthalate; and amide-based polymer such as nylon 6,6 and nylon 6. Examples of resin of the above resin-based sheet include, in addition to the resins that contain the above components, acetate resin, acrylonitrile-butadiene-styrene copolymer (ABS) resin, polystyrene resin, vinyl chloride resin, polyester amide resin, polyether resin, polyimide resin, polyamide imide resin, poly-p-phenylene sulfide resin, and polyether ester resin. The base material may contain one kind of these materials, or may also contain two or more kinds.

Among the above-described resin components of the resin-based sheet, olef in-based polymer may preferably be used. As used in the present description, the term "olefin-based polymer" means a homopolymer or a copolymer obtained by polymerizing one or more kinds selected from olefin monomers. Examples of the olefin monomers include olefin monomers having a carbon number of 2 to 8, α-olefin monomers having a carbon number of 3 to 18, and olefin monomers having a cyclic structure. Examples of the olefin monomers having a carbon number of 2 to 8 include ethylene, propylene, 2-butene, and octene. Examples of the α-olefin monomers include propylene, 1-butene, 4-methyl-1-pentene, 1-hexene, 1-octene, 1-decene, 1-dodecene, 1-tetradecene, and 1-octadecene. Examples of the olefin monomers having a cyclic structure include norbornene, cyclopentadiene, cyclohexadiene, dicyclopentadiene, and tetracyclododecene, and derivatives thereof.

When the olefin-based polymer contains polyethylene, the polyethylene may be any of high-density polyethylene, medium-density polyethylene, low-density polyethylene, ultralow-density polyethylene, and linear low-density polyethylene, and may also be a mixture of two or more kinds thereof.

Specific examples of the olefin-based polymer of copolymer include: polyethylene-based resin, such as ethylene-propylene copolymer, ethylene-vinyl acetate copolymer, ethylene-vinyl acetate-maleic anhydride copolymer, ethylene-(meth)acrylic acid copolymer, ethylene-butyl acrylate copolymer, and ethylene-(meth)acrylic ester-maleic anhydride copolymer; polypropylene-based resin, such as propylene-ethylene-diene compound copolymer; acid-modified polyolefin; and ionomer-based resin.

When the olefin-based polymer contains ethylene-propylene copolymer, the ethylene-propylene copolymer may be any of block polymer and random polymer, and may also be a mixture thereof.

One kind of the olefin-based polymer may be solely used, or two or more kinds may also be used in a mixture.

The base material may be composed of a single layer of the above resin-based sheet, or may also be composed of a multilayer obtained by laminating a plurality of the above resin-based sheets. When the base material comprises a resin-based sheet or resin-based sheets, the resin-based sheet or sheets may not be oriented, or may also be monoaxially or biaxially oriented such as in the longitudinal and/or lateral directions.

When the base material is composed of a resin-based material, the base material may be produced by coextrusion molding with the pressure sensitive adhesive layer.

Specific examples of the base material composed of paper-based material include paper base material, such as glassine paper, coated paper and wood-free paper, and laminated paper obtained by laminating the above paper base material with a thermoplastic resin, such as polyethylene.

The material that constitutes the base material may contain a coloring material (examples thereof include pigment, such as carbon black and titanium dioxide, and colorant) thereby to color the base material. In view of enhancing the mechanical properties and imparting the blocking resistance, the material that constitutes the base material may contain fine particles, such as silica. The base material may further contain components, such as filler, antioxidant and ultraviolet absorber, depending on the intended use.

The base material may be formed with a primer coat layer, printed layer, coating layer, metal deposition layer, ultraviolet prevention layer, etc.

The above primer coat layer may preferably be a layer that contains a styrene-based thermoplastic elastomer. The styrene-based thermoplastic elastomer may preferably be a non-perfectly hydrogenated styrene-based thermoplastic elastomer in which ethylenic double bonds are partially hydrogenated or hydrogenation is not performed. According to the feature that a layer that contains a styrene-based thermoplastic elastomer is formed as a primer coat on the base material, it is possible to effectively suppress the occurrence of delamination in the pressure sensitive adhesive tape according to the present embodiment. It is preferred herein to use, as the base material, the above-described resin-based sheet using an olefin-based polymer because in this case there is significantly exerted an effect of suppressing the occurrence of delamination owing to the layer, as the primer coat layer, which contains the styrene-based thermoplastic elastomer.

As used in the present description, the term "styrene-based thermoplastic elastomer" means a material comprising a copolymer that contains a constitutional unit derived from styrene or its derivatives (styrene-based compound) and having rubber-like elasticity in a temperature range including an ordinary temperature while having thermoplasticity. The term "non-perfectly hydrogenated styrene-based thermoplastic elastomer" as used herein means a styrene-based thermoplastic elastomer in which hydrogenation is not perfectly performed, and specific examples thereof include a non-hydrogenated styrene-based thermoplastic elastomer and a partially hydrogenated styrene-based thermoplastic elastomer.

Examples of the above styrene-based thermoplastic elastomer include styrene-conjugate diene copolymer. Specific examples of the styrene-conjugate diene copolymer include: non-hydrogenated styrene-conjugate diene copolymers, such as styrene-butadiene copolymer (SBR), styrene-butadiene-styrene copolymer (SBS), styrene-butadiene-butylene-styrene copolymer, styrene-isoprene copolymer, styrene-isoprene-styrene copolymer (SIS), and styrene-ethylene-isoprene-styrene copolymer, etc. Examples of the commercially available products in the industrial field include Tufprene (registered trademark) available from Asahi Kasei Corp., Kraton (registered trademark) available from Kraton Polymer Japan, Sumitomo TPE-SB available from Sumitomo Chemical Company, Limited, Epofriend (registered trademark) available from Daicel Corporation, Rabalon (registered trademark) available from Mitsubishi Chemical Corporation, Septon (registered trademark) available from KURARAY CO., LTD., and Tuftec (registered trademark) available from Asahi Kasei Corp.

Specific examples of the partially hydrogenated styrene-based thermoplastic elastomer include a partially hydrogenated product of styrene-butadiene copolymer (SBS) and a partially hydrogenated product of styrene-isoprene copolymer (SIS). The non-perfectly hydrogenated styrene-based thermoplastic elastomer contained in the primer coat layer may preferably comprise a partially hydrogenated product of SIS among the above products, and may more preferably consist of a partially hydrogenated product of SIS.

When the primer coat layer contains a non-perfectly hydrogenated styrene-based thermoplastic elastomer, the styrene-containing ratio in the non-perfectly hydrogenated styrene-based thermoplastic elastomer may preferably be 20 mass% or more and 80 mass% or less. According to the feature that the styrene-containing ratio is within the above range, it is possible to enhance the peel resistance of the primer coat layer to the base material and to the pressure sensitive adhesive layer, i.e., to reduce the possibility of occurrence of delamination in the pressure sensitive adhesive tape.

In view of more stably enhancing the peel resistance of the primer coat layer to the base material, the above styrene-containing ratio may preferably be 25 mass% or more, and more preferably 30 mass% or more.

In view of more stably enhancing the peel resistance of the primer coat layer to the pressure sensitive adhesive layer, the above styrene-containing ratio may preferably be 75 mass% or less, and more preferably 70 mass% or less.

The thickness of the primer coat layer is not particularly limited, but may ordinarily be 1 µm or more and 100 µm or less. If this thickness is unduly small, the productivity of the primer coat layer may possibly deteriorate, while unduly large thickness may possibly cause subsidiary problems, such as that the productivity deteriorates. The thickness of the primer coat layer may preferably be 2 µm or more and 50 µm or less, more preferably 4 µm or more and 40 µm or less, and furthermore preferably 6 µm or more and 20 µm or less.

The thickness of the base material should be appropriately set in accordance with the intended use, and is not limited. The thickness of the base material may ordinarily be 10 µm to 1,000 µm, preferably 20 µm to 500 µm, and particularly preferably about 25 µm to 200 µm. In the present description, the thickness of the base material means a thickness of those including layers, such as a primer coat layer, printed layer, coating layer, metal deposition layer, and ultraviolet prevention layer, which are formed on the base material.

The composition of the pressure sensitive adhesive layer of the pressure sensitive adhesive tape according to an embodiment of the present invention is not limited, provided that the pressure sensitive adhesive layer is formed of a pressure sensitive adhesive composition that contains a block copolymer of an acrylic-based monomer as the base polymer, and that the total amount of an unreacted monomer raw material contained in the pressure sensitive adhesive layer is 100 ppm or less.

The pressure sensitive adhesive composition for forming the pressure sensitive adhesive layer may be solid under an ordinary temperature, and the pressure sensitive adhesive layer may be produced by a production method that includes molding work of the pressure sensitive adhesive composition. The molding work may be injection molding or extrusion molding.

The pressure sensitive adhesive composition for forming the pressure sensitive adhesive layer may also be in a liquid-like form under an ordinary temperature. In this case, the liquid-like pressure sensitive adhesive composition may contain a cross-linking agent, such as an isocyanate-based compound and epoxy compound, in addition to the base polymer comprising a block copolymer of an acrylic-based monomer. When the liquid-like pressure sensitive adhesive composition contains a crosslinking agent, the pressure sensitive adhesive layer may contain a crosslinked product of the base polymer and the crosslinking agent.

The pressure sensitive adhesive layer may contain a coloring material (examples thereof include pigment, such as carbon black and titanium dioxide, and colorant) thereby to color the pressure sensitive adhesive layer. The pressure sensitive adhesive layer may further contain components, such as filler, antioxidant and ultraviolet absorber, depending on the intended use.

The thickness of the pressure sensitive adhesive layer should be appropriately set in accordance with the intended use, and is not limited. As a basic tendency, an increased thickness of the pressure sensitive adhesive layer may enhance the pressure sensitive adhesion of the pressure sensitive adhesive tape to an adherend, but the amount of migrated components from the pressure sensitive adhesive tape to an adherend is likely to increase. The thickness of the pressure sensitive adhesive layer may ordinarily be 1 µm to 1,000 µm, preferably 2 µm to 500 µm, more preferably 5 µm to 100 µm, and particularly preferably about 10 µm to 50 µm.

The pressure sensitive adhesive tape according to an embodiment of the present invention may be in an elongated form and wound up into a roll. In this case, the surface of the pressure sensitive adhesive layer of the pressure sensitive adhesive tape may be protected by the surface of the base material of the pressure sensitive adhesive tape opposite to the surface facing the pressure sensitive adhesive layer, until use.

In order to protect the surface of the pressure sensitive adhesive layer of the pressure sensitive adhesive tape according to an embodiment of the present invention, a release sheet may be attached to the surface of the pressure sensitive adhesive tape at the side of the pressure sensitive adhesive layer.

To an extent that does not impair the advantageous effects of the present invention, additives, such as non-crosslinkable resin, crosslinkable resin, tackifier, coupling agent, filler, softener, plasticizer, surfactant, antioxidant, heat stabilizer, light stabilizer, ultraviolet absorber, colorant, antifoam, flame retardant, and antistatic, may be added to the material that forms the above pressure sensitive adhesive layer.

The non-crosslinkable resin is effective for adjustment of viscosity and prevention of autohesion. It is preferred that the non-crosslinkable resin can be powder within a temperature region in the hopper of an extruder. It is also preferred that the non-crosslinkable resin has compatibility to the pressure sensitive adhesive layer. In view of more stably realizing the added resin having compatibility to the pressure sensitive adhesive layer, it is more preferred that the added resin is a resin of the same series as the pressure sensitive adhesive layer. That is, when the pressure sensitive adhesive layer comprises an acrylic acid-based resin, it is preferred that the added resin is also an acrylic acid-based resin. The content of the added resin in the pressure sensitive adhesive layer is not limited, provided that it functions to adjust the viscosity of the pressure sensitive adhesive layer. The content of the added resin in the pressure sensitive adhesive layer may preferably be more than 0.01 mass% and less than 10 mass%, more preferably 0.1 mass% or more and 5 mass% or less, and particularly preferably 0.5 mass% or more and 3 mass% or less.

Examples of the release sheet to be used include: films, such as polyethylene film, polypropylene film, polybutene film, polybutadiene film, polymethylpentene film, polyvinyl chloride film, vinyl chloride copolymer film, polyethylene terephthalate film, polyethylene naphthalate film, polybutylene terephthalate film, polyurethane film, ethylene vinyl acetate film, ionomer resin film, ethylene- (meth) acrylic acid copolymer film, ethylene-(meth)acrylic ester copolymer film, polystyrene film, polycarbonate film, polyimide film, and fluorine resin film; and papers, such as glassine paper and wood-free paper. A laminate film obtained by laminating a plurality of such films may also be used.

It is preferred that the release surface (surface to be in contact with the pressure sensitive adhesive layer) of the above release sheet is subjected to release treatment. Examples of a release agent to be used for the release treatment include alkyd-based, silicone-based, fluorine-based, unsaturated polyester-based, polyolefin-based, and wax-based release agents. The thickness of the release sheet is not particularly limited, and may ordinarily be about 20 µm to 150 µm.

The release sheet may be produced by molding work, such as extrusion molding. In this case, the release sheet may be formed by coextrusion molding together with the pressure sensitive adhesive layer.

The method of manufacturing the pressure sensitive adhesive tape according to an embodiment of the present invention is not limited. Examples of the method include, but are not limited to, methods as follows:

(Method 1) A pressure sensitive adhesive layer is formed on a base material by extrusion molding using a pressure sensitive adhesive composition for forming the pressure sensitive adhesive layer. If necessary, a release sheet may be attached to the surface of the obtained pressure sensitive adhesive layer, or alternatively a substance obtained by extrusion molding may be laminated between a base material and a release sheet.

(Method 2) A laminate of a pressure sensitive adhesive layer and a base material is formed on a release sheet by coextrusion molding using a pressure sensitive adhesive composition for forming the pressure sensitive adhesive layer and a resin composition for forming the base material.

(Method 3) A laminate of a pressure sensitive adhesive layer and a release sheet is formed on a base material by coextrusion molding using a pressure sensitive adhesive composition for forming the pressure sensitive adhesive layer and a resin composition for forming the release sheet.

(Method 4) A laminate of a base material, a pressure sensitive adhesive layer, and a release sheet is formed by coextrusion molding using a pressure sensitive adhesive composition for forming the pressure sensitive adhesive layer, a resin composition for forming the base material, and a resin composition for forming the release sheet.

(Method 5) A liquid-like pressure sensitive adhesive composition for forming a pressure sensitive adhesive layer is applied to a base material, and drying or the like is performed to form the pressure sensitive adhesive layer on the base material. If necessary, a release sheet may be attached to the surface of the obtained pressure sensitive adhesive layer.

(Method 6) A liquid-like pressure sensitive adhesive composition for forming a pressure sensitive adhesive layer is applied to a release sheet, and drying or the like is performed to form the pressure sensitive adhesive layer on the release sheet. A base material is attached to the surface of the obtained pressure sensitive adhesive layer.

In view of suppressing the TVOC (total volatile organic compounds), it is preferred that a solvent is not used in a film forming process. Therefore, any of the methods of manufacturing a pressure sensitive adhesive tape according to Methods 1 to 4 may be preferred.

When the above Methods 1 to 4 are employed, the pressure sensitive adhesive layer is produced by extrusion molding. In these cases, it is preferred that the pressure sensitive adhesive composition for forming the pressure sensitive adhesive layer contains a triblock copolymer. It is also preferred that the triblock copolymer satisfies at least one of a condition that the polystyrene equivalent weight-average molecular weight (Mw) is 30, 000 or more and 300, 000 or less and a condition that the molecular weight distribution (Mw/Mn) is 5.0 or less.

Any specific method of the above Methods 1 to 6 and conditions thereof (method of applying and conditions thereof, method of drying or the like and conditions thereof, method of extrusion molding and conditions thereof, and method of coextrusion molding and conditions thereof) may be appropriately set in accordance with the structure and use of the pressure sensitive adhesive tape to be manufactured (which may further comprise a release sheet), materials to be used for manufacturing the pressure sensitive adhesive tape (which may further comprise a release sheet), etc.

The embodiments heretofore explained are described to facilitate understanding of the present invention and are not described to limit the present invention. Therefore, it is intended that the elements disclosed in the above embodiments include all design changes and equivalents to fall within the technical scope of the present invention.

### [Examples]

Hereinafter, the present invention will be further specifically described with reference to examples, etc., but the scope of the present invention is not limited to these examples, etc. In the examples, Mw represents a polystyrene equivalent weight-average molecular weight, and Mn represents a polystyrene equivalent number-average molecular weight.

### [Example 1]

A pressure sensitive adhesive composition A was obtained as a material for forming a pressure sensitive adhesive layer by adding 0.5 weight parts of polymethyl methacrylate (PMMA) fine particles ("Techpolymer MB-4" available from Sekisui Plastics Co., Ltd., Mw=775,000) to 100 weight parts of pellet-like acrylic acid-based triblock pressure sensitive adhesive ("KURARITY LA3320" available from KURARAY CO., LTD., PMMA-polybutyl acrylate (PBA)-PMMA, PMMA ratio=18.7 mol%, Mw=119,000, Mw/Mn=1.1) and mixing them in a tumbler for 10 minutes.

The pressure sensitive adhesive composition A was put into an extruder, and film formation was performed using a T-die to form a pressure sensitive adhesive layer so that the thickness of the pressure sensitive adhesive layer would be 20 µm. The obtained pressure sensitive adhesive layer was laminated with a polyethylene terephthalate (PET) film ("LUMIRROR 50T60" available from Toray Industries, Inc., thickness of 50 µm) located to face one surface of the pressure sensitive adhesive layer and a release sheet ("SP-PET381031" available from LINTEC Corporation) located at the surface of the pressure sensitive adhesive layer opposite to the surface facing the PET film, and a pressure sensitive adhesive tape was thus obtained with the release sheet attached thereto.

### [Example 2]

A pressure sensitive adhesive composition B was obtained in the same manner as in Example 1 except that pellet-like acrylic triblock copolymer ("NABSTAR N800AS" available from KANEKA CORPORATION, PMMA-PBA-PMMA triblock copolymer, PMMA ratio=27.0 mol%, Mw=127,000, Mw/Mn=1.3), different from the material used in Example 1, was used as the pellet-like acrylic acid-based triblock pressure sensitive adhesive. The same operation as in Example 1 was performed using the pressure sensitive adhesive composition B, and a pressure sensitive adhesive tape was thus obtained with a release sheet attached thereto.

### [Comparative Example 1]

A reaction apparatus comprising a thermometer, a stirrer, a reflux condenser, and a nitrogen gas inlet was used and fed with 75 mass parts of toluene, 75 mass parts of ethyl acetate, 90 mass parts of butyl acrylate (BA), and 10 mass parts of acrylic acid (AA). After adding thereto 0.3 mass parts of azobisisobutyronitrile (AIBN), polymerization reaction was performed in a nitrogen gas atmosphere at 80°C for 8 hours, and BA-AA copolymer solution was obtained. Mw of the BA-AA copolymer was 500,000, and the mass ratio of a constitutional unit based on BA and a constitutional unit based on AA (BA:AA) was 90:10. Toluene was added to the obtained BA-AA copolymer solution to adjust the solid content to 34 mass%, and an acrylic-based copolymer solution C was thus obtained.

A liquid-like pressure sensitive adhesive composition D was produced by compounding 1.0 mass part of toluene diisocyanate (TDI, product name "BHS-8515" available from TOYO INK CO., LTD., solid content of 37.5 mass%) as an isocyanate-based crosslinking agent and 50 mass parts of toluene as a diluent solvent to 100 mass parts of the obtained acrylic copolymer solution C.

The above liquid-like pressure sensitive adhesive composition D was applied to the silicone resin release treatment surface of a release material ("SP-PET381031" available from LINTEC Corporation) using a knife-over-roll coater, and dried (90°C, 1 minute) to form a pressure sensitive adhesive layer (thickness of 30 µm). A PET film ("LUMIRROR 50T60" available from Toray Industries, Inc.) was attached to the surface of the obtained pressure sensitive adhesive layer with the release material, and a pressure sensitive adhesive tape was thus obtained with a release sheet attached thereto.

### [Comparative Example 2]

A reaction apparatus comprising a thermometer, a stirrer, a reflux condenser, and a nitrogen gas inlet was used and fed with 75 mass parts of toluene, 75 mass parts of ethyl acetate, 90 mass parts of 2-ethylhexyl acrylate (2EHA), and 10 mass parts of acrylic acid (AA). After adding thereto 0.3 mass parts of azobisisobutyronitrile (AIBN), polymerization reaction was performed in a nitrogen gas atmosphere at 80°C for 8 hours, and 2EHA-AA copolymer solution was obtained. Mw of the 2EHA-AA copolymer was 550,000, and the mass ratio of a constitutional unit based on 2EHA and a constitutional unit based on AA (2EHA:AA) was 90:10. Toluene was added to the obtained 2EHA-AA copolymer solution to adjust the solid content to 34 mass%, and an acrylic-based copolymer solution E was obtained. The same operation as in Comparative Example 1 was performed except that the obtained acrylic-based copolymer solution E was used as substitute for the acrylic-based copolymer solution C, and a pressure sensitive adhesive tape was thus obtained with a release sheet attached thereto.

### [Exemplary Test 1] Measurement of Total Amount of Monomer Raw Material

The release sheet was removed from each of the pressure sensitive adhesive tapes manufactured in the Examples and the Comparative Examples, and an evaluation sample was obtained by cutting the pressure sensitive adhesive tape so that the exposed surface of the pressure sensitive adhesive layer would have a surface area of 20 cm². The evaluation sample was enclosed in an ampule, which was then heated at 120 °C for 30 minutes in Purge & Trap GCMass ("JHS-100A" available from Japan Analytical Industry Co., Ltd.) to sample gasses, which were thereafter introduced into GCMass ("TurboMass" available from PerkinElmer), and the total amount of sampled monomer raw material was obtained as an n-decane equivalent amount (unit: ppm) with reference to a calibration curve prepared using n-decane. Results are listed in Table 1.

### [Exemplary Test 2] Measurement of Amount of Migrated Components

Test was conducted in accordance with "the basic rules necessary for testing migration of the constituents of plastic materials and articles intended to come into contact with foodstuffs" (Directive 82/711/EEC, 97/48/EC). The release sheet was removed from each of the pressure sensitive adhesive tapes manufactured in the Examples and the Comparative Examples, and a test sample was obtained by attaching the obtained pressure sensitive adhesive tape to a frame-shaped jig for exposing a predetermined size (surface area of 3 dm²) of the surface of the pressure sensitive adhesive layer. Subsequently, the test sample was stationarily placed at 20°C for 24 hours with the surface of the pressure sensitive adhesive layer of the pressure sensitive adhesive tape being in contact with 150 ml of an extraction solvent (95 vol% ethanol). Thereafter, only the extraction solvent was dried at 20°C, and drying operation was further performed at 120 °C for 30 minutes. The weight of residue after drying (unit: µg/cm²) was measured, and the measured value was to represent the amount of migrated components. As will be appreciated, 1 µg/cm² corresponds to 0.1 mg/dm². Results are listed in Table 1.

### [Exemplary Test 3] Measurement of Peel Strength

The release sheet was removed from each of the pressure sensitive adhesive tapes manufactured in the Examples and the Comparative Examples, and a pressure sensitive adhesive tape test piece was obtained by cutting the obtained pressure sensitive adhesive tape into a shape of 25 mm×150 mm. The above pressure sensitive adhesive tape test piece was attached to an adherend (SUS304 #600) to provide a test sample under conditions of 23°C and a relative humidity of 50% on the basis of JIS Z0237: 2009 (ISO 29862-29864: 2007). The peel strength (unit: N/25 mm) after 24 hours from the attaching was measured at a tensile speed of 300 mm/min using the 180° peeling test method. Results are listed in Table 1.

### [Exemplary Test 4] Measurement of Ball Tack

The release sheet was removed from each of the pressure sensitive adhesive tapes manufactured in the Examples and the Comparative Examples, and a pressure sensitive adhesive tape test piece was obtained by cutting the obtained pressure sensitive adhesive tape into a shape of 12 mm×150 mm. The ball tack value at a slope angle of 30° was measured under conditions of 23°C and a relative humidity of 50% on the basis of JIS Z0237: 2009 (ISO 29862-29864: 2007). Results are listed in Table 1.

### [Exemplary Test 5] Measurement of Holding Power

The release sheet was removed from each of the pressure sensitive adhesive tapes manufactured in the Examples and the Comparative Examples, and a pressure sensitive adhesive tape test piece was obtained by cutting the obtained pressure sensitive adhesive tape into a shape of 25 mm×150 mm. Based on JIS Z0237: 2009 (ISO 29862-29864: 2007), a load (9.8 N) was applied using a 1-kg weight under conditions of 23°C and a relative humidity of 50%, and the holding power was evaluated by measuring a period of time (holding time) from when the test was started to when the weight dropped off. Results are listed in Table 1. The testing time was 70, 000 seconds. The numerical value of "70, 000" in Table 1 means that the weight did not drop off even after the testing time of 70, 000 seconds passed, i.e., that the holding time was not less than 70, 000 seconds. Other numerical values than "70,000" each represent a period of time (unit: seconds) from when the test was started to when the weight dropped off.

**[Table 1]**

| | | Example 1 | Example 2 | Comparative Example 1 | Comparative Example 2 |
|---|---|---|---|---|---|
| Total Amount of Monomer Raw Material | (ppm) | <100 | <100 | 150 | 600 |
| Amount of Migrated Components | (µg/cm²) | 24 | 28 | 72 | 85 |
| Peel Strength | (N/25 mm) | 3.0 | 11.8 | 21.0 | 21.4 |
| Ball Tack | | 15 | 18 | 3 | 4 |
| Holding Power | | 70000 | 70000 | 70000 | 70000 |

### [Industrial Applicability]

The pressure sensitive adhesive tape according to the present invention can be suitably used as a pressure sensitive adhesive tape that is used in the fields, such as food and medical fields, for which high quality and sophisticated management system are required.

## Claims

1. A pressure sensitive adhesive tape comprising a base material and a pressure sensitive adhesive layer provided on one surface of the base material,
wherein the pressure sensitive adhesive layer is formed of a pressure sensitive adhesive composition that contains a block copolymer of an acrylic-based monomer as a base polymer,
wherein a total amount of an unreacted monomer raw material contained in the pressure sensitive adhesive layer is 100 ppm or less.

2. The pressure sensitive adhesive tape as recited in claim 1, wherein the block copolymer contains a triblock copolymer as a main component.

3. The pressure sensitive adhesive tape as recited in claim 2, wherein the triblock copolymer satisfies at least one of a condition that a polystyrene equivalent weight-average molecular weight (Mw) is 30, 000 or more and 300, 000 or less and a condition that a molecular weight distribution (Mw/Mn) is 5.0 or less.

4. The pressure sensitive adhesive tape as recited in any one of claims 1 to 3, wherein a peel strength in 180° peeling from a SUS plate (SUS304 #600) is 0.1 N/25 mm or more when measured in accordance with JIS Z0237: 2009.

5. The pressure sensitive adhesive tape as recited in any one of claims 1 to 4, wherein a ball stack value at a slope angle of 30° is 10 or more when measured in accordance with JIS Z0237: 2009.

6. The pressure sensitive adhesive tape as recited in any one of claims 1 to 5, wherein a holding time is 70,000 seconds or more when a holding power test is performed under conditions of a sample attaching area of 25 mm×25 mm, a load of 1 kg, and a temperature of 40°C in accordance with JIS Z0237: 2009.

7. The pressure sensitive adhesive tape as recited in any one of claims 1 to 6, wherein the pressure sensitive adhesive layer is produced by extrusion molding.
